## Europäisches Patentamt

(19) **European Patent Office**

**Office européen des brevets**

(11) Veröffentlichungsnummer: **0 102 498**
**B1**

(12) **EUROPÄISCHE PATENTSCHRIFT**

(45) Veröffentlichungstag der Patentschrift:
**07.01.87**

(21) Anmeldenummer: **83107251.7**

(22) Anmeldetag: **23.07.83**

(51) Int. Cl.⁴: **A 61 M 25/00**, A 61 M 5/14

(54) **Katheterset.**

(30) Priorität: **06.08.82 DE 8222222 U**

(43) Veröffentlichungstag der Anmeldung:
**14.03.84 Patentblatt 84/11**

(45) Bekanntmachung des Hinweises auf die Patenterteilung:
**07.01.87 Patentblatt 87/2**

(84) Benannte Vertragsstaaten:
**AT BE CH DE FR GB IT LI LU NL SE**

�titleEntgegenhaltungen:
**EP - A - 0 059 784**
**DE - A - 1 933 802**
**DE - B - 2 238 722**
**US - A - 3 090 384**
**US - A - 3 470 867**
**US - A - 3 506 007**

(73) Patentinhaber: **Mehler, Doron, Dr., Warburghof 18,**
**D-3000 Hannover 61 (DE)**
Patentinhaber: **Otten, Brigitte, Dr., Im Schiffsmoor 41,**
**D-2850 Bremerhaven-Surheide (DE)**

(72) Erfinder: **Mehler, Doron, Dr., Warburghof 18,**
**D-3000 Hannover 61 (DE)**
Erfinder: **Otten, Brigitte, Dr., Im Schiffsmoor 41,**
**D-2850 Bremerhaven-Surheide (DE)**

(74) Vertreter: **Junius, Walther, Dr., Wolfstrasse 24,**
**D-3000 Hannover 81 (DE)**

## Beschreibung

Die Erfindung betrifft einen Katheterset für die Plexus-Anästhesie.

Kathetersets werden auf verschiedenen Gebieten der Medizin zu verschiedenen Zwecken eingesetzt, jedoch nicht zur kontinuierlichen Plexus-Anästhesie.

Schon seit 1946 werden Methoden zur Plexus-Anästhesie über verschiedene Zugänge zum Plexus brachialis beschrieben. Dabei wurden bisher zum Erreichen dieser Leitungsanästhesie nur Hilfsmittel angewendet, wie Venen-Verweilkanülen, Epidural-Katheter mit TUOHY-Nadeln, Spinalnadeln mit darüberzogenem Teflon-Katheter oder auch Venenpunktions-Kanülen kombiniert mit Venen-Verweilkathetern. Nachteil aller dieser Methoden ist die Gefahr der Nerven- und/oder Gefässverletzung durch die scharfe Kanülenspitze. Auch die TUOHY-Nadel ist in dieser Hinsicht nicht ungefährlich.

Um diese Gefahr der Nerven- und/oder Gefässverletzung durch die scharfe Kanülenspitze zu vermeiden, ist für die Plexus-Anästhesie eine immobile Nadel von Zenz und Glocker entwickelt worden. Diese Nadel ermöglicht eine einmalige Injektion von Lokalanästhetikum, nach dessen Wirkungsverlust erneut punktiert werden muss. Der Wirkungsspiegel erreicht daher ein Maximum, dessen Abfall von den pharmakologischen Eigenschaften des zur Anwendung gebrachten Lokalanästhetikums abhängt. Diese Nadel eignet sich aufgrund ihrer Konzeption vorwiegend für die Analgesie respektive Anästhesie von maximal 6 bis 10 Stunden und findet daher eine überwiegende Verwendung im operativen Bereich. Die Kürze der Nadel bedingt einen relativ steilen Winkel zur neurovaskulären axillären Fascie mit der insbesondere bei Anfängern noch vorhandenen Gefahr der Nervenläsion und evtl. auch Gefässverletzungen. Die dünne Nadel perforiert die neurovaskuläre Fascie der Axilla mit einem nur leichten «click»-Phänomen und einem weniger deutlichen Widerstandsverlust bei stetig ausgeübtem Stempeldruck durch eine aufgesetzte und mit physiologischer NaCl-Lösung gefüllten Spritze. Mit dieser Nadel lässt sich ein Katheter nicht in die Nervengefässloge einführen.

Für das Einlegen von Kathetern sind auf verschiedenen anderen Gebieten der Medizin Kathetersets eingesetzt, jedoch nicht zur kontinuierlichen Plexus-Anästhesie.

Der Katheterset der US-A-35 06 007 wird eingesetzt, um durch Einführung einer von einem Katheter überzogenen Nadel senkrecht zu dessen Verlaufsrichtung in den Epiduralraum einzudringen. Diese Nadel weist einen Schliffwinkel von 45° auf, die Nadellänge beträgt 80 mm oder ist länger.

Unter dem gleichen Einbringungswinkel von 90° oder nahezu 90° ist für die Plexus-Anästhesie die immobile Nadel von Zenz und Glocker geschaffen, die aus diesem Grunde nur eine Länge von unter 30 mm hat.

Es ist die Aufgabe der Erfindung, auch für die Plexus-Anästhesie einen Katheterset zu schaffen, mit dem es selbst dem Ungeübten gelingt, ohne Nervenverletzung einen Katheter in die Nervengefässloge einzuführen und der bei einer geringmöglichen Traumatisierung eine praktisch unbegrenzte Wirkungsdauer gewährleistet.

Die Erfindung besteht in der Schaffung eines Kathetersets für die Plexus-Anästhesie, bestehend aus einer Spritze, einem daran zu befestigenden Mandrin, dessen Spitze unter einem Winkel von mehr als 40° zur Achsrichtung geschliffen ist und dessen Länge mindestens 50 mm beträgt, einer über den Mandrin gezogenen Kunststoffkanüle mit einem Kanülenansatz, der Vorsprünge zur Fixierung des Kanülenansatzes an der Fassung des Mandrins aufweist und einem durch die Kunststoffkanüle schiebbaren Katheter mit Spritzenansatz, wobei der Aussendurchmesser des Katheters etwas kleiner als der Innendurchmesser der Kanüle ist.

Dabei ist es zweckmässig, wenn Öffnungen im durch die Kunststoffkanüle schiebbaren Katheter versetzt angeordnet sind und wenn die Öffnungen bei einer Länge der Einführtiefe des Katheters von 8 bis 10 cm in den ersten 15 mm angeordnet sind.

Weiter ist es von erheblichem Vorteil, wenn das Mandrin zu beiden Seiten des am weitesten vorstehenden Punktes seiner Spitze an zwei Stellen seitlich angeschliffen ist.

Hierdurch werden folgende Vorteile erzielt:

1. Durch den relativ flachen Winkel der Kanüle zur neurovaskulären Fascie und den flachen Winkel des Schliffs an der Mandrinspitze sind relativ hohe Kräfte notwendig, um die Fascie zu perforieren. Dabei sind ähnlich wie bei der Periduralanästhesie der Widerstandsverlust deutlich fühlbar und ein «Click»-Phänomen oftmals deutlich hörbar. Im Vergleich zur Verwendung von kürzeren Nadeln sind beide Erscheinungen wesentlich ausgeprägter.

Eine Nerven- oder Gefässläsion ist wegen des flachen Punktionswinkels mit der speziellen Kanülenspitze unwahrscheinlich, da das Mandrin dieser Kanüle nach Perforation der Fascie auch weiterhin fast tangential zu den innerhalb der Fascie verlaufenden Gefässen und Nerven liegt. Ausserdem wird nach Perforation der Fascie der weiche Teflonanteil der Kanüle über das Mandrin in den neurovaskulären Raum vorgeschoben. Auch hierdurch wird die Gefahr der Nerven- und Gefässläsion vermieden.

2. Das Auslösen von Parästhesien ist bei Beachten dieses Vorgehens nicht nötig. Wie festgestellt werden konnte, eignet sich daher dieses Set auch besonders gut für Patienten, mit denen eine Kommunikation nicht möglich ist (z.B. Anästhesie bei Eingriffen der oberen Extremität bei alkoholisierten, nicht-nüchternen Patienten, Sympathikolyse bei Intensivpatienten etc.). Durch die schnelle Injektion von kalter physiologischer NaCl-Lösung lassen sich oftmals atraumatisch Parästhesien auslösen.

3. Durch die verminderte Gefahr der Gefässverletzung mit Hämatombildung und durch den längeren subcutanen Verlauf des Katheters bis zum perivaskulären Raum kann die Möglichkeit einer Infektion bzw. deren Ausbreitung in der Regio axillaris bei entsprechender Pflege (täglicher Verbandswechsel, Polyvidonjodsalbe) stark herabgesetzt werden. So musste bei einer durchschnittlichen Liegedauer von 14 bis 21 Tagen kein Katheter wegen Infektion entfernt werden. Die längste Verweildauer eines Katheters betrug 7 Wochen. Auch hier kam es nicht zur Infektion.

4. Für den Anfänger in der Anästhesie bietet sich der Vorteil, eine Intraneurale Injektion zu vermeiden, auf das Auslösen von Parästhesien verzichten zu können und mit sehr hoher Wahrscheinlichkeit auch den N. musculocutaneus mit zu analgesieren bzw. mit zu anästhesieren.

Das Wesen der Erfindung ist nachstehend anhand eines in der Zeichnung schematisch dargestellten Ausführungsbeispieles näher erläutert. Es zeigen:

Fig. 1 eine Ansicht des auseinandergenommenen Kathetersets,

Fig. 2 eine perspektivische Ansicht der Spitze des Mandrins.

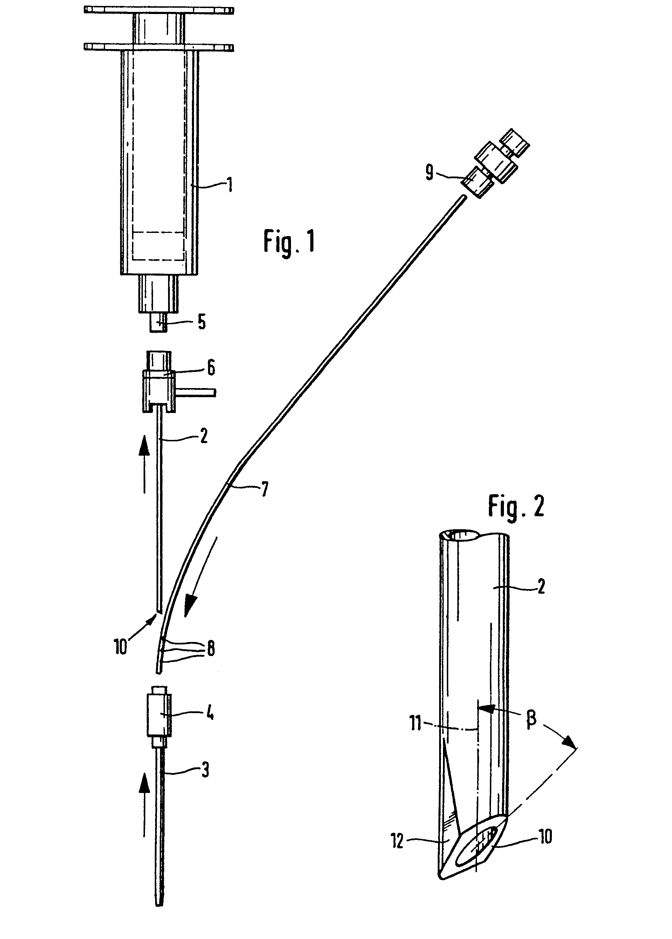

In Fig. 1 sind die Teile des Kathetersets in auseinandergenommenen Zustand dargestellt. Für die Anwendung werden die einzelnen Teile zu einem einheitlichen Ganzen, das dann den Gebrauchsgegenstand bildet, zusammengesteckt.

Auf die Spritze 1 wird ein Mandrin 2 aufgesteckt, über das die Kunststoffkanüle 3 mit dem Kanülenansatz gesteckt wird. Dazu wird über den Ansatz 5 an der Spritze 1 die Fassung 6 geschoben, in der das Mandrin 2, das die Form einer dünnen hohlen Metallnadel hat, steckt. Die Kunststoffkanüle 3 ist mit dem Kanülenansatz 4 versehen, der einen wesentlich weiteren Durchmesser hat und Vorsprünge trägt. Diese Vorsprünge dienen der Fixierung des Kanülenansatzes 4 an der Fassung 6 des Mandrins. Durch die Kunststoffkanüle 3 ist das Katheter 7 hindurchschiebbar, welches an seinem vorderen Ende Öffnungen 8 aufweist, die zweckmässigerweise gegeneinander versetzt angeordnet sind. An dem Katheter anschliessbar ist ein Spritzenansatz 9.

Sehr wichtig ist der Schliff der Spitze 10 des Mandrins 2 unter einem Winkel β von mindestens 40° gegenüber der Achse 11 des Mandrins 2. Sehr vorteilhaft ist ein seitliches Anschleifen des vorderen Teiles des Mandrins, so dass hier die Materialstärke des Mandrins vermindert ist. Das erleichtert die Einführung des Mandrins ohne die Gefahr einer Nervenbeschädigung.

**Patentansprüche**

1. Katheterset für die Plexusanästhesie, bestehend aus einer Spritze (1), einem daran zu befestigen Mandrin (2), dessen Spitze (10) unter einem Winkel von mehr als 40° zur Achsrichtung geschliffen ist und dessen Länge mindestens 50 mm beträgt, einer über den Mandrin gezogenen Kunststoffkanüle (3) mit einem Kanülenansatz (4), der Vorsprünge zur Fixierung des Kanülenansatzes (4) an der Fassung (6) des Mandrins aufweist, und einem durch die Kunststoffkanüle schiebbaren Katheter (7) mit Spritzenansatz (9), wobei der Aussendurchmesser des Katheters etwas kleiner als der Innendurchmesser der Kanüle ist.

2. Katheterset nach Anspruch 1, dadurch gekennzeichnet, dass Öffnungen (8) im durch die Kunststoffkanüle (3) verschiebbaren Katheter (8) versetzt angeordnet sind und dass die Öffnungen bei einer Länge der Einführtiefe des Katheters (8) von 8 bis 10 cm in den ersten 15 mm angeordnet sind.

3. Katheterset nach Anspruch 1 und 2, dadurch gekennzeichnet, dass das Mandrin (2) zu beiden Seiten des am weitesten vorstehenden Punktes seiner Spitze an zwei Stellen seitlich angeschliffen ist.

**Revendications**

1. Ensemble formant cathéter pour l'anesthésie des plexus, constitué d'une seringue (1), d'un mandrin (2) à y fixer, dont la pointe (10) est taillée sous un angle de plus de 40° par rapport à sa direction axiale et dont la longueur atteint au moins 50 mm, d'une canule en plastique (3) qui est tirée par dessus le mandrin et comporte un embout (4) qui présente des saillies pour la fixation de l'embout (4) de la canule sur la monture (6) du mandrin, et d'un cathéter (7) qui peut coulisser à travers la canule de plastique et comporte un embout (9) de raccordement à la seringue, le diamètre extérieur du cathéter étant un peu inférieur au diamètre intérieur de la canule.

2. Ensemble formant cathéter selon la revendication 1, caractérisé en ce que les ouvertures (8) sont disposées, décalées, dans le cathéter (8) qui peut coulisser à travers la canule de plastique (3); et en ce que, dans le cas d'une longueur de profondeur d'introduction du cathéter (8) de 8 à 10 cm, les ouvertures sont disposées dans les premiers 15 mm.

3. Ensemble formant cathéter selon la revendication 1 et la revendication 2, caractérisé en ce que le mandrin (2) est taillé latéralement, en deux endroits, des deux côtés du point de sa pointe qui est le plus en avant.

**Claims**

1. A catheter assembly for plexus anaesthesia consisting of a syringe (1), a mandrin (2), the tip (10) of which is cut at an angle of more than 40° to the axial direction and the length of which is at least 50 mm, a synthetic resin cannula (3) which is drawn over the mandrin and has a cannula attachment (4) which has projections for fixing it to the mounting (6) of the mandrin, and a catheter (7) which can be pushed through the synthetic resin cannula and has a syringe attachment (9), the outer diameter of the catheter being somewhat smaller than the inner diameter of the cannula.

2. A catheter assembly as claimed in Claim 1, characterised in that openings (3) are arranged in staggered manner in the catheter (8) which can be moved through the synthetic resin cannula (3), and that for a length of the insertion depth of the catheter (8) of 8 to 10 cm, the openings are arranged within the first 15 mm.

3. A catheter assembly as claimed in Claim 1 and 2, characterised in that on both sides of the furthest protruding point of its tip the mandrin (2) is laterally ground at two points.

Fig. 1

Fig. 2